(19) **Europäisches Patentamt** / **European Patent Office** / **Office européen des brevets**

(11) **EP 2 499 967 B1**

(12) **EUROPEAN PATENT SPECIFICATION**

(45) Date of publication and mention
of the grant of the patent:
**25.06.2014 Bulletin 2014/26**

(21) Application number: **10829860.5**

(22) Date of filing: **01.11.2010**

(51) Int Cl.:
***A61B 5/02*** *(2006.01)*     ***A61B 5/0245*** *(2006.01)*

(86) International application number:
**PCT/JP2010/069408**

(87) International publication number:
**WO 2011/058900 (19.05.2011 Gazette 2011/20)**

(54) **PULSE WAVE PROPAGATION SPEED MEASUREMENT DEVICE AND PULSE WAVE PROPAGATION SPEED MEASUREMENT PROGRAM**

VORRICHTUNG ZUR MESSUNG DER GESCHWINDIGKEIT DER PULSWELLENAUSBREITUNG UND PROGRAMM ZUR MESSUNG DER GESCHWINDIGKEIT DER PULSWELLENAUSBREITUNG

DISPOSITIF DE MESURE DE VITESSE DE PROPAGATION D'ONDES PULSÉES ET PROGRAMME DE MESURE DE VITESSE DE PROPAGATION D'ONDES PULSÉES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **10.11.2009 JP 2009257217**

(43) Date of publication of application:
**19.09.2012 Bulletin 2012/38**

(73) Proprietor: **Sharp Kabushiki Kaisha
Osaka-shi, Osaka 545-8522 (JP)**

(72) Inventors:
• **IKEDA, Yutaka
 Osaka-shi, Osaka 545/8522 (JP)**

• **HORI, Atsushi
 Osaka-shi, Osaka 545/8522 (JP)**

(74) Representative: **Treeby, Philip David William
RGC Jenkins & Co.
26 Caxton Street
London SW1H 0RJ (GB)**

(56) References cited:
**EP-A2- 1 273 264**     **JP-A- 2003 010 139**
**JP-A- 2003 199 718**     **JP-A- 2005 349 116**
**JP-A- 2007 007 075**

**Description**

TECHNICAL FIELD

The present invention relates to a pulse wave velocity measurement device and a pulse wave velocity measurement program for measuring pulse waves of a living body and thereby calculating velocities at which the pulse waves are propagated.

BACKGROUND ART

It has conventionally been known that pulse waves have various important information for grasp on a state of a circulatory system of a living body. In particular, a velocity and time for propagation of a pulse wave between two sites in a living body are living-body indices that have drawn attention from the medical work front because a possibility has been pointed out that a state of arteriosclerosis may be grasped by the indices, and are called Pulse Wave Velocity (PWV) and Pulse Transit Time (PTT), respectively, or the like.

A plurality of measurement methods according to measurement sites have been proposed for the pulse wave velocity, and the pulse wave velocity between a carotid artery and a femoral artery, for instance, is called cfPWV (carotid-femoral PWV) and is used as a gold standard of the pulse wave velocity (PWV).

In general, two measurement points are required in order to find the pulse wave velocity (PWV).

It is known, however, that a pulse wave measured at any site on a living body is a resultant wave of an ejection wave from the heart and reflected waves reflected from various sites in the living body. There is a possibility that separation into the ejection wave and the reflected waves may make it possible to find the pulse wave velocity or the pulse transit time even if only one measurement point for pulse waves exists.

In Non-Patent Literature 1, therefore, a technique for separating the ejection wave and the reflected waves is disclosed. In Patent Literature 1 and Patent Literature 2, there are disclosed techniques for finding the pulse wave velocity or the pulse transit time by separating the ejection wave and the reflected waves even in presence of only one measurement point for pulse waves.

In general, reflection of pulse waves occurs at various sites in a living body. The reflection of pulse waves is caused mainly by impedance mismatch in blood vessels and thus occurs at sites having blood vessel bifurcation, variation in elastic force in blood vessel or the like, for instance.

As described in Patent Literature 1, the separation into the ejection wave and the reflected waves is satisfactorily attained for pulse waves measured at various sites in a living body, on an assumption that a staple reflection point is in vicinity of iliac arteries or an abdominal aorta.

Disclosed in Patent Literature 2 is a technique for calculating the pulse wave velocity or the pulse transit time and finding a blood pressure from pulse waves obtained at one measurement point on basis of a correlation between the pulse wave velocity or the pulse transit time and the blood pressure. Disclosed in Non-Patent Literature 2 is a technique for calculating the pulse wave velocity or the pulse transit time and finding a blood pressure from pulse waves obtained at two measurement points.

It is disclosed in both Patent Literatures 1 and 2 described above that the pulse wave velocity or the pulse transit time can be found on basis of pulse waves at the one measurement point, and this is based on a premise that the pulse wave velocities of the ejection wave and the reflected waves are equal. According to the premise, (1) one pulse wave is separated into an ejection wave and a reflected wave and a time difference between both the waves is found, and (2) a difference between pulse wave propagation distances of the ejection wave and the reflected wave is found. The pulse wave velocity or the pulse transit time is calculated therefrom.

The pulse wave velocities of the ejection wave and the reflected wave, however, do not actually coincide with each other. That is because amplitudes of the ejection wave and the reflected wave differ. As noted in Patent Literature 2 described above, the pulse wave velocity is correlated with the blood pressure, which relates to the amplitude of the pulse wave. That is, the pulse wave velocity relates to the amplitude of the pulse wave.

As described above, a waveform of a pulse wave can satisfactorily be explained on assumption that the reflection point of the reflected wave is in vicinity of the abdominal aorta. Though the reflection of pulse waves is caused by the impedance mismatch in aortas and the abdominal aorta, degrees of the mismatch differ among individuals and according to physical conditions and conditions of blood vessels in one individual. As a result, the amplitude of the reflected wave differs from that of the traveling wave, and degrees of the difference differ among individuals. This results in a difference in the pulse wave velocity between the ejection wave and the reflected wave.

CITATION LIST

PATENT LITERATURE

Patent Literature 1: JP 2003-010139 A

Patent Literature 2: JP 2007-007075 A

Non-Patent Literature 1: Takazawa K et al. "Underestimation of vasodilator effects of nitroglycerin by upper limb blood pressure", Hypertension 1995; 26:520-3

Non-Patent Literature 2: McCombie, Devin "Development of a wearable blood pressure monitor using adaptive calibration of peripheral pulse transit time measurements", Ph.D. Thesis, Massachusetts Institute of Technology, Dept. of Mechanical Engineering, 2008.

EP 1,273,264 discloses the subject-matter of the preambles of claims 1 and 4.

SUMMARY OF INVENTION

Technical Problem

Therefore, an object of the invention is to provide a pulse wave velocity measurement device and a pulse wave velocity measurement program by which a pulse wave velocity can more accurately be found on basis of pulse waves at one measurement site.

Solution to Problem

According to the present invention there is provided a pulse wave velocity measurement device according to claim 1.
In a pulse wave velocity measurement device according to one embodiment, the pulse wave amplitude detection unit includes an ejection wave component elimination unit as defined in claim 2.
According to the embodiment, the ejection wave component elimination unit eliminates the ejection wave component from the amplitude of the pulse wave that corresponds to the reference time for the reflected wave component and thereby finds the amplitude of the reflected wave component that corresponds to the reference time for the reflected wave component, so that the amplitude of the reflected wave component that corresponds to the reference time for the reflected wave component can accurately be found. Therefore, the velocity of the pulse wave can accurately be found by the pulse wave velocity detection unit.
According to a second aspect of the present invention there is provided a pulse wave velocity measurement program according to claim 4. amplitudes of the pulse wave that correspond to the reference times for the identification of the ejection wave component and the reflected component. Thus the pulse wave velocity can be detected with higher accuracy in consideration of the difference between the pulse wave velocities of the ejection wave and the reflected wave which difference is caused by a difference between the amplitude of the ejection wave component and the amplitude of the reflected wave component.
In a pulse wave velocity measurement program according to one embodiment, the pulse wave amplitude deriving function includes an ejection wave component eliminating function as defined in claim 5.
According to the pulse wave velocity measurement program of the embodiment, the ejection wave component included in the amplitude of the pulse wave is eliminated by the ejection wave component eliminating function from the amplitude of the pulse wave that corresponds to the reference time for the reflected wave component, and the amplitude of the reflected wave component that corresponds to the reference time for the reflected wave component is thereby found. Thus the amplitude of the reflected wave component that corresponds to the reference time for the reflected wave component can more accurately be found. Therefore, the velocity of the pulse wave can more accurately be found by the pulse wave velocity deriving function.
In order that the present invention be more readily understood specific embodiments thereof will now be described.

BRIEF DESCRIPTION OF DRAWINGS

Fig. 1 is a block diagram of a pulse wave velocity measurement device that is an embodiment of the invention;

Fig. 2A is a waveform chart showing a waveform of a pulse wave detected by a pulse wave detection unit in the embodiment;

Fig. 2B is a waveform chart showing a waveform of an acceleration wave of the pulse wave;

Fig. 2C is a waveform chart showing a waveform of a third derivative of the pulse wave detected by the pulse wave detection unit in the embodiment;

Fig. 2D is a waveform chart showing a waveform of a fourth derivative of the pulse wave;

Fig. 3 is a waveform chart showing a waveform of the pulse wave and amplitudes W1, W2 of the pulse wave at reference points Q1, Q2 of the pulse wave;

Fig. 4 is a waveform chart showing a waveform of the pulse wave, and an ejection wave component S1 and a reflected wave component S2 of the pulse wave; and

Fig. 5 is a schematic diagram that schematically shows a path d through which the ejection wave component S1 of the pulse wave S propagates in a human living body and a path h through which the reflected wave component S2 propagates in the human living body.

DESCRIPTION OF EMBODIMENTS

Hereinbelow, the invention will be described in detail with reference to an embodiment shown in the drawings.

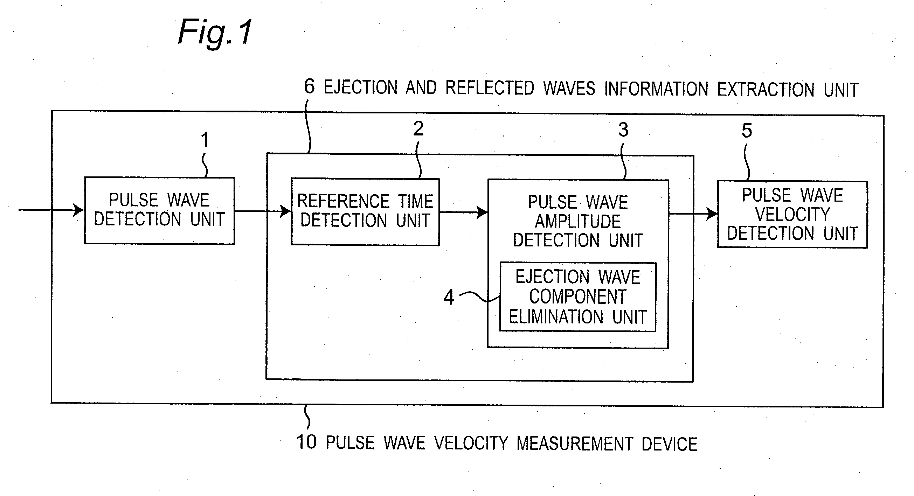

Fig. 1 is a block diagram showing a pulse wave velocity measurement device 10 that is an embodiment of the invention. The pulse wave velocity measurement device 10 includes a pulse wave detection unit 1 that detects pulse waves at one site in a living human body. The pulse wave detection unit 1 may be, for instance, a unit in which a degree of reflection or absorption of infrared light outputted from a light emitting device according to a quantity of blood in a blood vessel is measured by a light receiving device (photoplethysmography). Alternatively, the pulse wave detection unit 1 may be a unit that detects pulse waves by pressure pulse wave method in which a change in pressure of blood in a blood vessel against the blood vessel is extracted as electric signals. Especially severe limitations do not exist on the site in a living body where pulse waves are detected by the pulse wave detection unit 1, whereas the site is desirably set at a site noninvasive and unconstrained, if possible, and is preferably set on a finger tip, a wrist, an earlobe or the like.

The pulse wave velocity measurement device 10 includes an ejection and reflected waves information extraction unit 6 for detecting reference times for identification of an ejection wave component and a reflected wave component that are included in the pulse wave detected by the pulse wave detection unit 1 and amplitudes of the pulse wave that correspond to the reference times, and a pulse wave velocity detection unit 5 for finding a velocity of the pulse wave on basis of information representing the reference time and the amplitudes that is sent from the ejection and reflected waves information extraction unit 6.

The ejection and reflected waves information extraction unit 6 includes a reference time detection unit 2 for detecting the reference time for the identification of the ejection wave component included in the pulse wave detected by the pulse wave detection unit 1 and the reference time for identification of the reflected wave component included in the pulse wave.

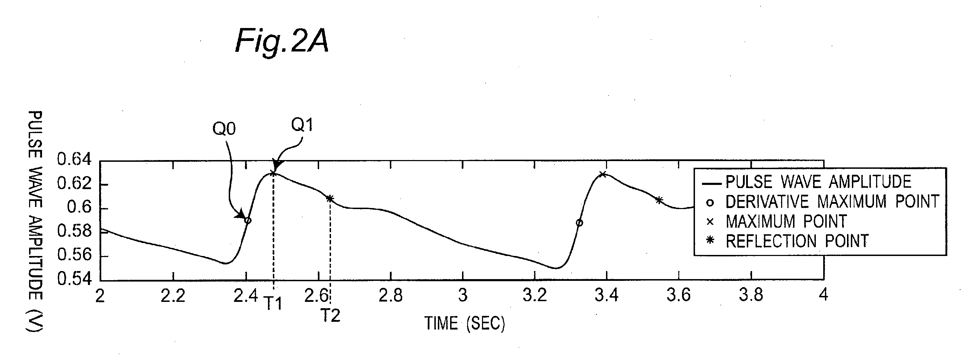

An example of a process in which the reference time detection unit 2 finds the reference times will be described below. Fig. 2A shows an example of a waveform of a pulse wave detected by the pulse wave detection unit 1. A vertical axis in Fig. 2A represents measured voltage values (V) corresponding to amplitudes (mmHg) of the pulse wave. In the pulse wave velocity measurement device 10 of the embodiment, correction (calibration) of correspondence of the voltage values (V) measured by the pulse wave detection unit 1 with the amplitudes (mmHg) of the pulse wave is performed on basis of blood pressures (mmHg) measured by a conventional cuff-type sphygmomanometer, as an example. The correction (calibration) has only to be performed when first use thereof is started, and a result of the calibration has only to be used for the subsequent measurement.

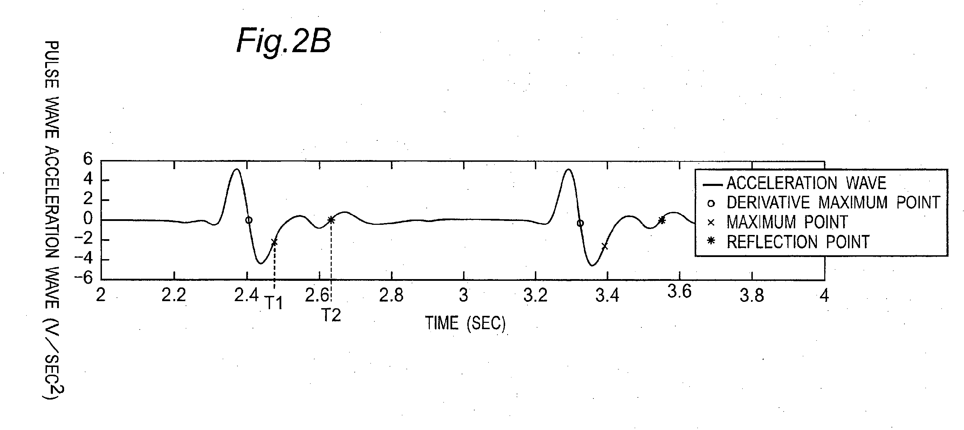

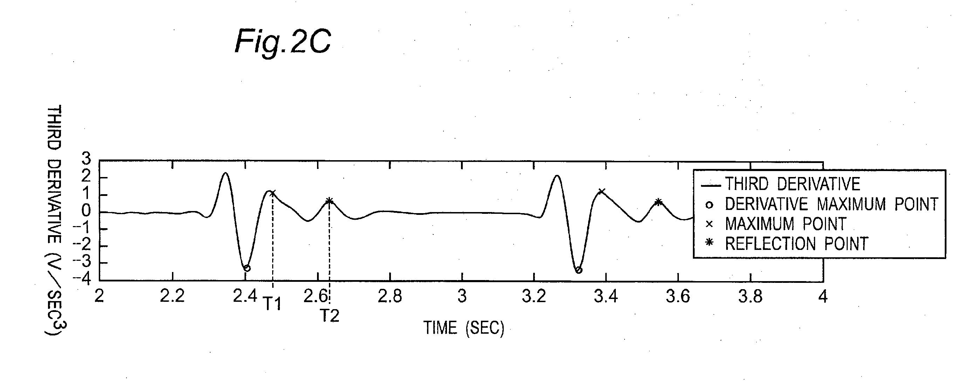

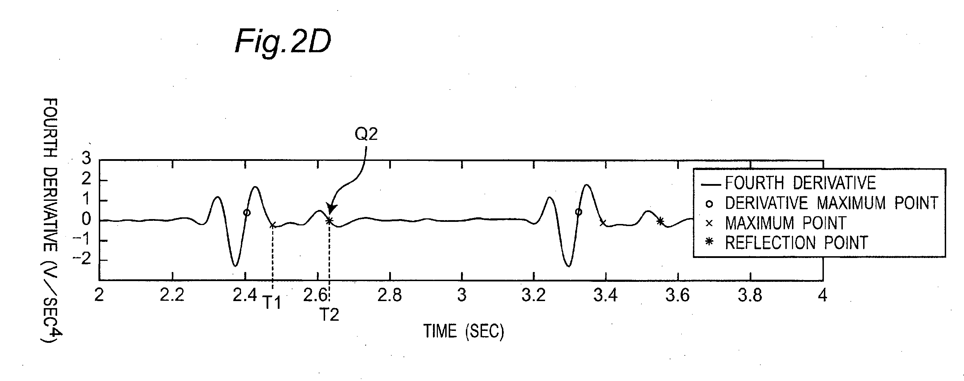

On condition that the pulse wave is of Type C in blood pressure waveform classification by Murgo et al., for instance, the reference time detection unit 2 detects time T1, at a maximum point Q1 in a systole in a waveform of the pulse wave shown in Fig. 2A, as reference time T1 for the ejection wave component. Fig. 2B shows an acceleration wave of the pulse wave, and Fig. 2C shows a third derivative of the pulse wave. On condition that the pulse wave is of Type C in the blood pressure waveform classification by Murgo et al., for instance, the reference time detection unit 2 detects a third zero cross point Q2 of a fourth derivative of the pulse wave shown in Fig. 2D, as reference time T2 for the reflected wave component. The third zero cross point Q2 signifies a point where the fourth derivative waveform shown in Fig. 2D crosses zero downward at the third time after the pulse wave shown in Fig. 2A passes the minimum value.

Though the above description has been given on the detected pulse wave that is of Type C in the blood pressure waveform classification, a method by which the reference time for the ejection wave component of the pulse wave and the reference time for the reflected wave component thereof can more preferably be determined may be employed, if any, on condition that the detected pulse waves have a waveform other than Type C in the blood pressure waveform classification. For instance, basically, pulse waves do not exhibit so great change in waveform unless there is great fluctuation in the blood pressure, and thus an accuracy in detecting a pulse wave can be improved by superimposition (arithmetic mean) of a plurality of measured pulse waves.

Pulse waves exhibit various waveforms according to noise levels, ages, sex, presence or absence of illness, physical conditions, and the like, and thus a method by which the reference time for the ejection wave component of the pulse waves and the reference time for the reflected wave component thereof can more preferably be determined may be employed. For instance, an accuracy in determining the reference time for the ejection wave component of the pulse wave and the reference time for the reflected wave component thereof can be improved by recording, as a history, of the detected pulse waves along with conditions of a measured person at that point of time.

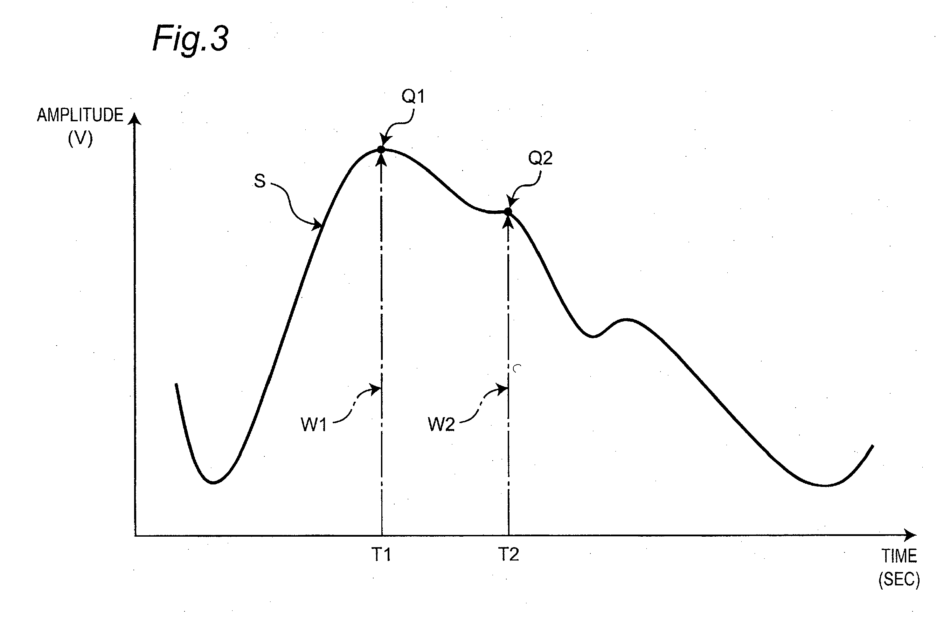

The ejection and reflected waves information extraction unit 6 includes a pulse wave amplitude detection unit 3. As shown in a waveform diagram of Fig. 3 as an example, the pulse wave amplitude detection unit 3 detects an amplitude W1 of the pulse wave S that corresponds to the reference time T1 for the ejection wave component detected by the reference time detection unit 2 and detects an amplitude W2 of the pulse wave S that corresponds to the reference time T2 for the reflected wave component detected by the reference time detection unit 2.

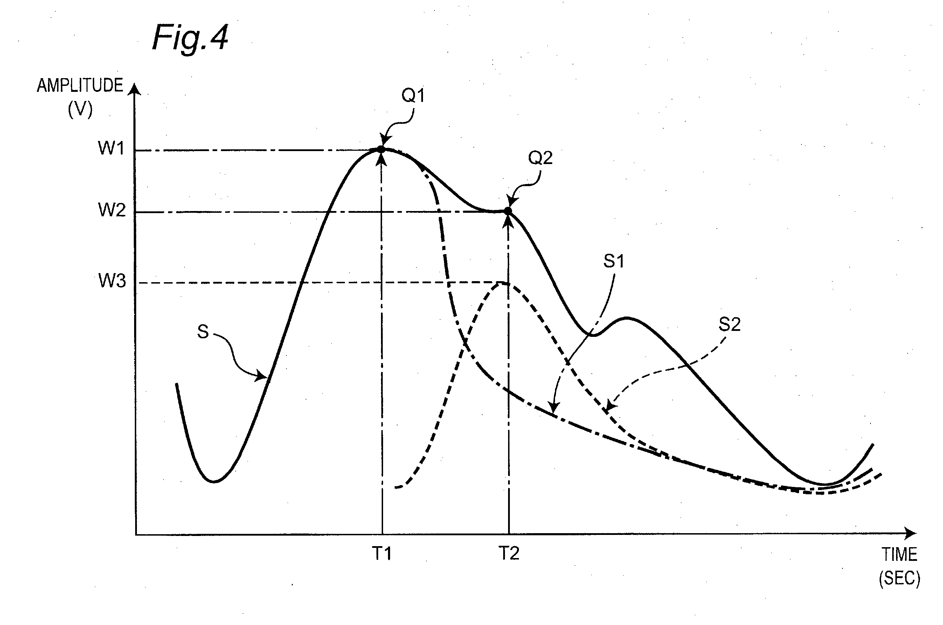

The ejection and reflected waves information extraction unit 6 further includes an ejection wave component elimination unit 4. As shown in Fig. 4, the ejection wave component elimination unit 4 eliminates an ejection wave component S1 of the pulse wave S from the amplitude W2 of the pulse wave S, which amplitude corresponds to the reference time T2 for the reflected wave component S2 and thereby finds an amplitude W3 of the reflected wave component S2, that corresponds to the reference time T2 for the reflected wave component S2. As a method of finding the amplitude W3 of the reflected wave component S2 that corresponds to the reference time T2 for the reflected wave component S2, a method is conceivable in which degrees of decrease in the ejection wave in the pulse wave are modeled with use of Windkessel model, for instance, and in which a remaining ejection wave component S1 is subtracted from the pulse wave amplitude measured around the reference time T2 for the reflected wave component S2. As a matter of course, a method by which the remaining ejection wave component S1 can more accurately be determined may be employed, if any.

The ejection and reflected waves information extraction unit 6 inputs into the pulse wave velocity detection unit 5 information representing the reference time T1 for the ejection wave component and the reference time T2 for the reflected wave component detected by the reference time detection unit 2 and inputs into the pulse wave velocity detection unit 5 information representing the amplitude W1 of the pulse wave S and the amplitude W3 of the reflected wave component S2 of the pulse wave S that have been detected by the pulse wave amplitude detection unit 3 and that correspond to the reference time T1 for the ejection wave component and the reference time T2 for the reflected wave components, respectively. Then the pulse wave velocity detection unit 5 finds the velocity of the pulse wave S on basis of the reference time T1 for the ejection wave component, the reference time T2 for the reflected wave component, the amplitude W1 of the pulse wave S that corresponds to the reference time T1 for the ejection wave component, and the amplitude W3 of the reflected wave component S2 of the pulse wave S that corresponds to the reference time T2 for the reflected wave component S2.

Subsequently, a process in which the pulse wave velocity detection unit 5 finds the pulse wave velocity PWV of the pulse wave S will be described.

For the pulse wave velocity, in general, pulse waves at two sites in a living body are measured and a velocity at which the ejection wave component of the pulse waves is propagated is found by a fundamental principle that is based on the principle of Moens-Korteweg. In Non-Patent Literature 2, a relation between the pulse wave velocity and the blood pressure is derived from the relation of Moens-Korteweg and is expressed as the following equation (1).

$$(PWV)^2 = \alpha \times exp(\beta \times P) \qquad\qquad (1)$$

In the equation (1), PWV is the pulse wave velocity (m/sec), P is the blood pressure (mmHg), and $\alpha$ and $\beta$ are constants that slightly change among individuals and according to measurement time even in an individual.

Application of the equation (1) to the pulse wave velocity PWV1 (m/sec) of the ejection wave S1 and the pulse wave velocity PWV2 (m/sec) of the reflected wave S2 provides the following equations (2) and (3), respectively.

$$(PWV1)^2 = \alpha \times exp(\beta \times W1) \qquad\qquad (2)$$

$$(PWV2)^2 = \alpha \times \exp(\beta \times W3) \qquad (3)$$

In the equation (2), W1 is the pulse wave amplitude of the ejection wave S1, that is, the amplitude W1 of the pulse wave S that corresponds to the reference time T1 for the ejection wave S1 of Fig. 4, and the amplitude W1 corresponds to a pulse wave pressure of the ejection wave S1. W3 therein is the pulse wave amplitude of the reflected wave S2, that is, the amplitude W3 of the reflected wave S2 that corresponds to the reference time T2 for the reflected wave S2 of Fig. 4, and the amplitude W3 corresponds to a pulse wave pressure of the reflected wave S2.

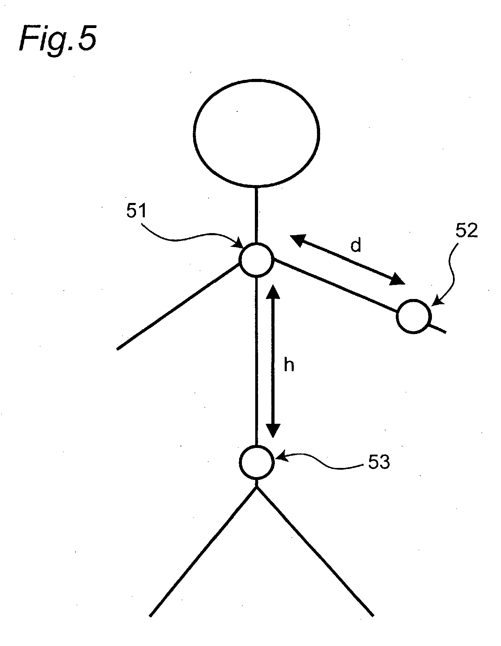

The following equations (4) and (5) are obtained with a distance from a heart 51 of a human body to a pulse wave measurement site 52 designated by d (m), a distance from the heart 51 to a reflection point 53 designated by h (m), a time period between a pulsation of the heart 51 and the reference time T1 for the ejection wave S1 designated by $\Delta$T1 (sec), and a time difference (T2-T1) between the reference time T1 for the ejection wave S1 and the reference time T2 for the reflected wave S2 designated by $\Delta$T2 (sec), as shown in Fig. 5.

$$(PWV1)^2 = (d/\Delta T1)^2 \qquad (4)$$

$$(PWV2)^2 = ((2h+d)/(\Delta T1+\Delta T2))^2 \qquad (5)$$

The following equation (6) is obtained from the equations (2) and (4) and the following equation (7) is obtained from the equations (3) and (5).

$$\alpha \times \exp(\beta \times W1) = (d/\Delta T1)^2 \qquad (6)$$

$$\alpha \times \exp(\beta \times W3) = ((2h+d)/(\Delta T1+\Delta T2))^2 \qquad (7)$$

The time $\Delta$T1 can be eliminated from the equations (6) and (7). That is, the following equation (8) is obtained from the equation (7).

$$(\Delta T1+\Delta T2)^2 = (2h+d)^2/\alpha \times \exp(\beta \times W3)$$

$$\Delta T1+\Delta T2 = (2h+d)/\{\alpha \times \exp(\beta \times W3)\}^{1/2}$$

$$\Delta T1 = (2h+d)/\{\alpha \times \exp(\beta \times W3)\}^{1/2} - \Delta T2 \qquad (8)$$

Substitution of the equation (8) for the equation (4) provides the following equation (9).

$$(PWV1)^2 = d^2 \times [(2h+d)/\{\alpha \times \exp(\beta \times W3)\}^{1/2} - \Delta T2]^{-2}$$

$$PWV1 = d \times [(2h+d)/\alpha \times \exp(\beta \times W3)]^{1/2} - \Delta T2]^{-1} \qquad (9)$$

That is, the pulse wave velocity PWV1 of the ejection wave S1 can be calculated from the known constants $\alpha$, $\beta$, the distances d, h that are known measured values, the difference (T2-T1) = $\Delta$T2 between the reference times T2 and T1 that are found by the reference time detection unit 2, and the amplitude W3 of the reflected wave S2, found by the ejection wave component elimination unit 4, that corresponds to the reference time T2 for the reflected wave S2.

Namely, the pulse wave velocity detection unit 5 finds the pulse wave velocity PWV1 of the ejection wave S1 of the pulse wave S, by the equation (9) derived from the equations (4) through (7) described above, on basis of the difference $\Delta T2$ between the reference times T1 and T2 for the ejection wave component S1 and the reflected component S2, and the amplitudes W1, W3 of the ejection wave component S1 and the reflected component S2 of the pulse wave S. Thus the pulse wave velocity can be measured with high accuracy in consideration of a difference between the pulse wave velocities of the ejection wave and the reflected wave which difference is caused by a difference between the amplitude W1 of the ejection wave component S1 and the amplitude W3 of the reflected wave component S2.

The embodiment in which the pulse wave amplitude detection unit 3 includes the ejection wave component elimination unit 4 has been described above, whereas the following equation (10) in which the amplitude W3 of the reflected wave S2 that corresponds to the reference time T2 for the reflected wave S2 in the equation (3) is replaced by the amplitude W2 of the pulse wave S that corresponds to the reference time T2 for the reflected wave S2 is used providing that the pulse wave amplitude detection unit 3 does not include the ejection wave component elimination unit 4.

$$(PWV2)^2 = \alpha \times \exp(\beta \times W2) \qquad\qquad (10)$$

Under that condition, the following equation (11) is obtained from the equations (5) and (10).

$$\alpha \times \exp(\beta \times W2) = ((2h+d)/(\Delta T1 + \Delta T2))^2 \qquad (11)$$

Therefore, the following equation (12) is obtained by elimination of the time $\Delta T1$ from the above described equation (6) and the equation (11).

$$\Delta T1 = (2h+d)/\{\alpha \times \exp(\beta \times W2)\}^{1/2} - \Delta T2 \qquad (12)$$

Substitution of the equation (12) into the above described equation (4) provides the following equation (13).

$$(PWV1)^2 = d^2 \times [(2h+d)/\{\alpha \times \exp(\beta \times W2)\}^{1/2} - \Delta T2]^{-2}$$

$$PWV1 = d \times [(2h+d)/\alpha \times \exp(\beta \times W2)]^{1/2} - \Delta T2]^{-1} \qquad (13)$$

That is, the pulse wave velocity PWV1 of the ejection wave S1 can be calculated from the known constants $\alpha$, $\beta$, the distances d, h that are the known measured values, the difference (T2-T1) = $\Delta T2$ between the reference times T2 and T1 that are found by the reference time detection unit 2, and the amplitude W2 of the pulse wave S that corresponds to the reference time T2 for the reflected wave S2 found by the pulse wave amplitude detection unit 3.

Namely, the pulse wave velocity detection unit 5 finds the pulse wave velocity PWV1 of the ejection wave S1 of the pulse wave S, by the equation (13) derived as described above, on basis of the difference $\Delta T2$ between the reference times T1 and T2 for the ejection wave component S1 and the reflected component S2, and the amplitude W2 of the pulse wave S at the reference time T2 that corresponds to the reference time T2 for the reflected wave component S2. Thus the pulse wave velocity can be measured with high accuracy in consideration of the difference between the pulse wave velocities of the ejection wave S1 and the reflected wave S2 of the pulse wave S.

Though the device for measuring the pulse wave velocity has been described for the embodiment, a blood pressure can be measured on basis of the pulse wave velocity measured in the embodiment, as apparent from the equation (1). That is, the pulse wave velocity found by the pulse wave velocity measurement device may be displayed as a blood pressure, instead of being displayed as it is, when a user uses the pulse wave velocity measurement device. That is because the blood pressure is a living-body index that is more familiar to people other than medical personnel than the pulse wave velocity though the pulse wave velocity is a living-body index that is extremely familiar to medical personnel.

A conventional sphygmomanometer using a cuff presents to a user a set composed of a systolic blood pressure, a diastolic blood pressure, a mean blood pressure, a pulse rate, and the like with use of several tens of seconds, whereas a sphygmomanometer based on the embodiment of the invention is capable of detecting a blood pressure for each pulsation and thus brings about a possibility that conditions of a living body can more minutely be grasped. Besides, the pulse wave velocity and the blood pressure can be detected thereby with higher accuracy by acquisition of an arithmetic mean, a moving average and/or the like of a unit of several pulsations.

By a pulse wave velocity measurement program, a computer may be made to execute a reference time deriving function of the reference time detection unit 2 finding the reference times T1 and T2, a pulse wave amplitude deriving function of the pulse wave amplitude detection unit 3 finding the amplitudes W1, W2 of the pulse wave S that correspond to the reference times T1, T2, an ejection wave component eliminating function of the ejection wave component elimination unit 4 finding the amplitude W3 of the reflected wave component S2 that corresponds to the reference time T2 for the reflected wave component S2, a function of the pulse wave velocity detection unit 5 finding the pulse wave velocity PWV1 of the ejection wave S1 of the pulse wave S on basis of the difference $\Delta T2$ between the reference times T1 and T2 for the ejection wave component S1 and the reflected component S2 and the amplitudes W3 of the reflected component S2 of the pulse wave S that corresponds to the reference time T2 for the reflected wave component S2, as described above.

By a pulse wave velocity measurement program, the computer may be made to execute the reference time deriving function of the reference time detection unit 2 finding the reference times T1 and T2, the pulse wave amplitude deriving function of the pulse wave amplitude detection unit 3 finding the amplitudes W1, W2 of the pulse wave S that correspond to the reference times T1, T2, and a function of the pulse wave velocity detection unit 5 finding the pulse wave velocity PWV1 of the ejection wave S1 of the pulse wave S on basis of the difference $\Delta T2$ between the reference times T1 and T2 for the ejection wave component S1 and the reflected component S2 and the amplitude W2 of the pulse wave S that corresponds to the reference time T2 for the reflected wave component S2, as described above.

REFERENCE SIGNS LIST

    1 pulse wave detection unit
    2 reference time detection unit
    3 pulse wave amplitude detection unit
    4 ejection wave component elimination unit
    5 pulse wave velocity detection unit 5
    6 ejection and reflected waves information extraction unit
    10 pulse wave velocity measurement device
    Q1 maximum point
    Q2 third zero cross point of fourth derivative
    S pulse wave
    S1 ejection wave
    S2 reflected wave
    T1 reference time for ejection wave component
    T2 reference time for reflected wave component
    W1 amplitude of pulse wave S that corresponds to reference time T1
    W2 amplitude of pulse wave S that corresponds to reference time T2
    W3 amplitude of reflected wave S2 that corresponds to reference time T2
    PWV1 pulse wave velocity of ejection wave S1
    PWV2 pulse wave velocity of reflected wave S2

**Claims**

**1.** A pulse wave velocity measurement device comprising:

    a pulse wave detection unit (1) for detecting a pulse wave at one site in a living body;
    a reference time detection unit (2) for detecting reference time for identification of an ejection wave component included in the pulse wave at the one site that is detected by the pulse wave detection unit (1) and reference time for identification of a reflected wave component included in the pulse wave;
    a pulse wave amplitude detection unit (3) for detecting an amplitude of the pulse wave that corresponds to the reference time for the ejection wave component detected by the reference time detection unit (2) and detecting an amplitude of the pulse wave that corresponds to the reference time for the reflected wave component detected by the reference time detection unit; and
    a pulse wave velocity detection unit (5) for finding a velocity of the pulse wave at which the pulse wave propagates, **characterized in that** the pulse wave velocity detection unit (5) finds the velocity of the pulse wave, on basis of the reference time for the ejection wave component and the reference time for the reflected wave component that have been detected by the reference time detection unit (2) and the amplitude of the pulse wave that corresponds to the reference time for the ejection wave component and the amplitude of the pulse wave that corresponds to the reference time for the reflected wave component which amplitudes have been detected by the pulse wave amplitude detection unit (3) by the following equation (1);

$$PWV1 = d \times [(2h+d)/\alpha \times exp(\beta \times W2)]1/2 - \Delta T2] - 1 \qquad (1)$$

where PWV1 is a pulse wave velocity of the ejection wave; $\alpha$, $\beta$ are known constants defined by the relation $(PWV)^2 = \alpha \times exp(\beta \times P)$ between the pulse wave velocity PWV and the blood pressure P; d, h are measured values of the distances from the heart and the reflection point of the reflected wave, respectively, to the one site; $\Delta T2$ is a time difference between the reference time for the ejection wave and the reference time for the reflected wave that are found by the reference time detection unit (2); and W2 is an amplitude of the pulse wave that corresponds to the reference time for the reflected wave found by the pulse wave amplitude detection unit (3).

2.  The pulse wave velocity measurement device as claimed in Claim 1, wherein
    the pulse wave amplitude detection unit (3) includes an ejection wave component elimination unit (4) for finding an ejection wave component included in the amplitude of the pulse wave that corresponds to the reference time for the reflected wave component, eliminating the ejection wave component from, the amplitude of the pulse wave that corresponds to the reference time for the reflected wave component, and thereby finding an amplitude of the reflected wave component that corresponds to the reference time for the reflected wave component, and wherein
    the pulse wave velocity detection unit (5) finds the velocity of the pulse wave on basis of the reference time for the ejection wave component and the reference time for the reflected wave component that have been detected by the reference time detection unit (2), the amplitude of the pulse wave that corresponds to the reference time for the ejection wave component that has been detected by the pulse wave amplitude detection unit (3), and the amplitude of the reflected wave component that has been found by the ejection wave component elimination unit (4) by the following equation (2) instead of equation (1);

$$PWV1 = d \times [(2h+d)/\alpha \times exp(\beta \times W3)]1/2 - \Delta T2] - 1 \qquad (2)$$

where W3 is an amplitude of the reflected wave that corresponds to the reference time for the reflected wave found by the ejection wave component elimination unit (4).

3.  The pulse wave velocity measurement device as claimed in Claim 2, wherein
    the pulse wave velocity detection unit (5) finds the velocity of the pulse wave on basis of a time difference between the reference time for the ejection wave component and the reference time for the reflected wave component that have been detected by the reference time detection unit (2), and an amplitude difference between the amplitude of the pulse wave that corresponds to the reference time for the ejection wave component and that has been detected by the pulse wave amplitude detection unit (3) and the amplitude of the reflected wave component that has been found by the ejection wave component elimination unit (4).

4.  A pulse wave velocity measurement program that makes a computer execute:

    a reference time deriving function of finding reference time for identification of an ejection wave component included in a pulse wave at one site in a living body and reference time for identification of a reflected wave component included in the pulse wave;
    a pulse wave amplitude deriving function of finding an amplitude of the pulse wave that corresponds to the reference time for the ejection wave component and finding an amplitude of the pulse wave that corresponds to the reference time for the reflected wave component; and
    a pulse wave velocity deriving function of calculating a velocity of the pulse wave at which the pulse wave propagates,
    **characterized in that**

the pulse wave velocity deriving function comprises calculating the velocity of the pulse wave, on basis of the reference time for the ejection wave component, the reference time for the reflected wave component, the amplitude of the pulse wave that corresponds to the reference time for the ejection wave component, and the amplitude of the pulse wave that corresponds to the reference time for the reflected wave component by the following equation (1);

$$\mathrm{PWV1} = d \times [(2h+d)/\alpha \times \exp(\beta \times W2)]1/2 - \Delta T2] - 1 \qquad (1)$$

where PWV1 is a pulse wave velocity of the ejection wave; $\alpha$, $\beta$ are known constants defined by the relation $(\mathrm{PWV})^2 = \alpha \times \exp(\beta \times P)$ between the pulse wave velocity, PWV and the blood pressure P; d, h are measured values of the distances from the heart and the reflection point of the reflected wave, respectively, to the one site; $\Delta T2$ is a time difference between the reference time for the ejection wave and the reference time for the reflected wave that are found by the reference time detection unit (2); and W2 is an amplitude of the pulse wave that corresponds to the reference time for the reflected wave found by the pulse wave amplitude detection unit (3).

**5.** The pulse wave velocity measurement program as claimed in Claim 4, wherein
the pulse wave amplitude deriving function includes an ejection wave component eliminating function of finding an ejection wave component included in the amplitude of the pulse wave that corresponds to the reference time for the reflected wave component, eliminating the ejection wave component included in the amplitude of the pulse wave from the amplitude of the pulse wave that corresponds to the reference time for the reflected wave component, and thereby finding an amplitude of the reflected wave component that corresponds to the reference time for the reflected wave component, and wherein
the pulse wave velocity deriving function comprises calculating the velocity of the pulse wave on basis of the reference time for the ejection wave component, the reference time for the reflected wave component, the amplitude of the pulse wave that corresponds to the reference time for the ejection wave component, and the amplitude of the reflected wave component that has been found by the ejection wave component eliminating function by the following equation (2) instead of equation (1);

$$\mathrm{PWV1} = d \times [(2h+d)/\alpha \times \exp(\beta \times W3)]1/2 - \Delta T2] - 1 \qquad (2)$$

where W3 is an amplitude of the reflected wave that corresponds to the reference time for the reflected wave found by the ejection wave component elimination unit (4).

**6.** The pulse wave velocity measurement program as claimed in Claim 5, wherein
the pulse wave velocity deriving function comprises finding the velocity of the pulse wave on basis of a time difference between the reference time for the ejection wave component and the reference time for the reflected wave component, and an amplitude difference between the amplitude of the pulse wave that corresponds to the reference time for the ejection wave component and the amplitude of the reflected wave component that has been found by the ejection wave component eliminating function.

## Patentansprüche

**1.** Pulswellengeschwindigkeitsmesseinrichtung,
mit:

einer Pulswellendetektionseinheit (1) zum Detektieren einer Pulswelle an einer Stelle eines lebenden Körpers,
einer Referenzzeitdetektionseinheit (2) zum Detektieren einer Referenzzeit für die Identifikation einer Ausgabewellenkomponente, welche in der Pulswelle enthalten ist, welche durch die Pulswellendetektionseinheit (2) an der einen Stelle detektiert wird, sowie einer Referenzzeit zur Identifikation einer reflektierten Wellenkomponente, die in der Pulswelle enthalten ist,
einer Pulswellenamplitudendetektioneinheit (3) zum Detektieren einer Amplitude der Pulswelle, welche zu der Referenzzeit für die Ausgabewellenkomponente korrespondiert, welche durch die Referenzzeitdetektionseinheit (2) detektiert wurde, und zum Detektieren einer Amplitude der Pulswelle, welche mit der Referenzzeit für die reflektierte Wellenkomponente korrespondiert, welche durch die Referenzzeitdetektionseinheit detektiert wurde, und

einer Pulswellengeschwindigkeitsdetektionseinheit (5) zum Auffinden einer Geschwindigkeit der Pulswelle, mit welcher sich die Pulswelle ausbreitet,

**dadurch gekennzeichnet,**

**dass** die Pulswellenwellengeschwindigkeitsdetektionseinheit (5) die Geschwindigkeit der Pulswelle auf der Grundlage der Bezugszeit für die Ausgabewellenkomponente und der Bezugszeit für die reflektierte Wellenkomponente auffindet, welche durch die Referenzzeitdetektionseinheit (2) detektiert wurden, sowie der Amplitude der Pulswelle, welche zu der Referenzzeit für die Ausgabewellenkomponente korrespondiert, und der Amplitude für die Pulswelle, welche zu der Referenzzeit für die reflektierte Wellenkomponente korrespondiert, wobei die Amplituden durch die Pulswellenamplitudendetektionseinheit (3) detektiert wurden, und zwar mittels der folgenden Gleichung (1):

$$PWV1 = d \times [(2h+d) / \alpha \times \exp(\beta \times W2)]1/2 - \Delta T2]\text{-}1, \qquad (1)$$

wobei PWV1 eine Pulswellengeschwindigkeit der Ausgabewelle bezeichnet, $\alpha$, $\beta$ bekannte Konstanten sind, die definiert sind durch die Beziehung

$$(PWV)^2 = \alpha \times \exp(\beta \times P)$$

zwischen der Pulswellengeschwindigkeit PWV und dem Blutdruck P, d, h Messwerte der Abstände vom Herzen bzw. vom Reflexionspunkt der reflektierten Welle in Bezug auf die eine Stelle sind, $\Delta T2$ eine Zeitdifferenz bezeichnet zwischen der Referenzzeit für die Ausgabe der Welle und der Referenzzeit für die reflektierte Welle, die durch die Referenzzeitdetektionseinheit (2) bestimmt wurden, und W2 eine Amplitude der Pulswelle bezeichnet, die zu der Referenzzeit für die reflektierte Welle korrespondiert, die durch die Pulswellenamplitudendetektionseinheit (3) detektiert wurde.

2. Pulswellengeschwindigkeitsmesseinrichtung nach Anspruch 1,
   wobei
   die Pulswellenamplitudendetektionseinheit (3) eine Ausgabewellenkomponenteneliminierungseinheit (4) aufweist zum Auffinden einer Ausgabewellenkomponente, welche in der Amplitude der Pulswelle enthalten ist, welche zu der Referenzzeit für die reflektierte Wellenkomponente korrespondiert, zum Eliminieren der Ausgabewellenkomponente von der Amplitude der Pulswelle, welche zu der Referenzzeit für die reflektierte Wellenkomponente korrespondiert, und dadurch Auffinden einer Amplitude der reflektierten Wellenkomponente, welche zu der Referenzzeit für die reflektierte Wellenkomponente korrespondiert, und
   die Pulswellengeschwindigkeitsdetektionseinheit (5) die Geschwindigkeit der Pulswelle ermittelt auf der Grundlage der Referenzzeit für die Ausgabewellenkomponente und der Referenzzeit für die reflektierte Wellenkomponente, welche durch die Referenzzeitdetektionseinheit (2) detektiert wurden, der Amplitude der Pulswelle, welche zu der Referenzzeit für die Ausgabewellenkomponente korrespondiert, welche durch die Pulswellenamplitudendetektionseinheit (3) detektiert wurde, und der Amplitude der reflektierten Wellenkomponente, welche durch die Ausgabewellenkomponenteneliminierungseinheit (4) aufgefunden wurde, und zwar mittels der folgenden Gleichung (2) anstelle der Gleichung (1):

$$PWV1 = d \times [(2h+d) / \alpha \times \exp(\beta \times W3)]1/2 - \Delta T2]\text{-}1, \qquad (2)$$

wobei W3 eine Amplitude der reflektierten Welle bezeichnet, welche zu der Referenzzeit für die reflektierte Welle korrespondiert, welche durch die Ausgabewellenkomponenteneliminierungseinheit (4) aufgefunden wurde.

3. Pulswellengeschwindigkeitsmesseinrichtung nach Anspruch 2,
   wobei die Pulswellengeschwindigkeitsdetektionseinheit (5) die Geschwindigkeit der Pulswelle auffindet auf der Grundlage einer Zeitdifferenz zwischen der Referenzzeit für die Ausgabewellenkomponente und der Referenzzeit für die reflektierte Wellenkomponente, welche durch die Referenzzeitdetektionseinheit (2) detektiert wurden, und einer Amplitudendifferenz zwischen der Amplitude der Pulswelle, welche zur Referenzzeit für die Ausgabewellenkomponente korrespondiert und welche durch die Pulswellenamplitudendetektionseinheit (3) detektiert wurde, und der Amplitude der reflektierten Wellenkomponente, welche durch die Ausgabewellenkomponenteneliminierungseinheit (4) aufgefunden wurde.

4. Pulswellengeschwindigkeitsmessprogramm, welches einen Computer dazu bringt auszuführen:

eine Referenzzeitableitungsfunktion zum Auffinden einer Referenzzeit für die Identifikation einer Ausgabewellenkomponente, die in der Pulswelle enthalten ist, an einer Stelle eines lebenden Körpers und einer Referenzzeit zur Identifikation einer reflektierten Wellenkomponente, die in der Pulswelle enthalten ist,
eine Pulswellenamplitudenableitungsfunktion des Auffindens einer Amplitude der Pulswelle, welche zu der Referenzzeit für die Ausgabewellenkomponente korrespondiert, die durch die Referenzzeitdetektionseinheit (2) detektiert wurde, und zum Auffinden einer Amplitude der Pulswelle, welche zu der Referenzzeit für die reflektierte Wellenkomponente korrespondiert, und
einer Pulswellengeschwindigkeitsableitungsfunktion des Berechnens einer Geschwindigkeit der Pulswelle, mit welcher sich die Pulswelle ausbreitet,
**dadurch gekennzeichnet,**
**dass** die Pulswellenwellengeschwindigkeitsableitungsfunktion das Berechnen der Geschwindigkeit der Pulswelle aufweist auf der Grundlage der Referenzzeit für die Ausgabewellenkomponente, der Referenzzeit für die reflektierte Wellenkomponente, der Amplitude der Pulswelle, welche zu der Referenzzeit für die Ausgabewellenkomponente korrespondiert, und der Amplitude für die Pulswelle, welche zu der Referenzzeit für die reflektierte Wellenkomponente korrespondiert, und zwar mittels der folgenden Gleichung (1):

$$PWV1 = d \times [(2h+d) / \alpha \times \exp (\beta \times W2)]1/2 - \Delta T2]\text{-}1, \qquad (1)$$

wobei PWV1 eine Pulswellengeschwindigkeit der Ausgabewelle bezeichnet, $\alpha$, $\beta$ bekannte Konstanten sind, die definiert sind durch die Beziehung

$$(PWV)^2 = \alpha \times \exp(\beta \times P)$$

zwischen der Pulswellengeschwindigkeit PWV und dem Blutdruck P, d, h Messwerte der Abstände vom Herzen bzw. vom Reflexionspunkt der reflektierten Welle in Bezug auf die eine Stelle sind, $\Delta T2$ eine Zeitdifferenz bezeichnet zwischen der Referenzzeit für die Ausgabe der Welle und der Referenzzeit für die reflektierte Welle, die durch die Referenzzeitdetektionseinheit (2) bestimmt wurden, und W2 eine Amplitude der Pulswelle bezeichnet, die zu der Referenzzeit für die reflektierte Welle korrespondiert, die durch die Pulswellenamplitudendetektionseinheit (3) detektiert wurde.

5. Pulswellengeschwindigkeitsmessprogramm nach Anspruch 4,
wobei
die Pulswellenamplitudendetektionsfunktion eine Ausgabewellenkomponenteneliminierungsfunktion aufweist zum Auffinden einer Ausgabewellenkomponente, welche in der Amplitude der Pulswelle enthalten ist, welche zu der Referenzzeit für die reflektierte Wellenkomponente korrespondiert, zum Eliminieren der Ausgabewellenkomponente von der Amplitude der Pulswelle, welche zu der Referenzzeit für die reflektierte Wellenkomponente korrespondiert, und dadurch Auffinden einer Amplitude der reflektierten Wellenkomponente, welche zu der Referenzzeit für die reflektierte Wellenkomponente korrespondiert, und
die Pulswellengeschwindigkeitsdetektionsfunktion das Berechnen der Geschwindigkeit der Pulswelle auf der Grundlage der Referenzzeit für die Ausgabewellenkomponente, der Referenzzeit für die reflektierte Wellenkomponente, der Amplitude der Pulswelle, welche zu der Referenzzeit für die Ausgabewellenkomponente korrespondiert, und der Amplitude der reflektierten Wellenkomponente, welche mittels der Ausgabewellenkomponenteneliminierungsfunktion aufgefunden wurde, aufweist, und zwar mittels der folgenden Gleichung (2) anstelle der Gleichung (1):

$$PWV1 = d \times [(2h+d) / \alpha \times \exp (\beta \times W3)]1/2 - \Delta T2]\text{-}1, \qquad (2)$$

wobei W3 eine Amplitude der reflektierten Welle bezeichnet, welche zu der Referenzzeit für die reflektierte Welle korrespondiert, die durch die Ausgabewellenkomponenteneliminierungseinheit (4) aufgefunden wurde.

6. Pulswellengeschwindigkeitsmessprogramm nach Anspruch 5,
wobei
wobei die Pulswellengeschwindigkeitsdetektionsfunktion das Auffinden der Geschwindigkeit der Pulswelle aufweist auf der Grundlage einer Zeitdifferenz zwischen der Referenzzeit für die Ausgabewellenkomponente und der Referenzzeit für die reflektierte Wellenkomponente und einer Amplitudendifferenz zwischen der Amplitude der Pulswelle, welche zur Referenzzeit für die Ausgabewellenkomponente korrespondiert, und der Amplitude der reflektierten Wellenkomponente, welche durch die Ausgabewellenkomponenteneliminierungsfunktion aufgefunden wurde.

**Revendications**

1. Dispositif de mesure de vitesse d'ondes pulsées, comprenant:

une unité de détection d'onde pulsée (1) pour détecter une onde pulsée à un endroit d'un corps vivant ;
une unité de détection de temps de référence (2) pour détecter un temps de référence pour l'identification d'une composante d'onde d'éjection contenue dans l'onde pulsée à l'endroit qui est détecté par l'unité de détection d'onde pulsée (1), et un temps de référence pour l'identification d'une composante d'onde réfléchie contenue dans l'onde pulsée ;
une unité de détection d'amplitude d'onde pulsée (3) pour détecter une amplitude de l'onde pulsée qui correspond au temps de référence pour la composante d'onde d'éjection détectée par l'unité de détection de temps de référence (2) et pour détecter une amplitude de l'onde pulsée qui correspond au temps de référence pour la composante d'onde réfléchie détectée par l'unité de détection de référence ; et
une unité de détection de vitesse d'onde pulsée (5) pour trouver une vitesse de l'onde pulsée à laquelle l'onde pulsée se propage,
**caractérisé en ce que** l'unité de détection de vitesse d'onde pulsée (5) trouve la vitesse de l'onde pulsée, sur la base du temps de référence pour la composante d'onde d'éjection et du temps de référence pour la composante d'onde réfléchie qui ont été détectés par l'unité ce détection de temps de référence (2), et de l'amplitude de l'onde pulsée qui correspond au temps de référence pour la composante d'onde d'éjection et de l'amplitude de l'onde pulsée qui correspond au temps de référence pour la composante d'onde réfléchie, lesquelles amplitudes ont été détectées par l'unité de détection d'amplitude d'onde pulsée (3), par l'équation (1) suivante :

$$PWV1 = d \times [(2h+d) / \alpha \times \exp(\beta x W2)]1/2-\Delta T2]-1 \qquad (1)$$

dans laquelle PWV1 désigne une vitesse d'onde pulsée de l'onde d'éjection ; $\alpha$, $\beta$ des constantes connues définies par la relation $(PWV)^2 = \alpha$ x exp ($\beta$xP) entre la vitesse d'onde pulsée PWV et la pression artérielle P ; d, h des valeurs mesurées des distances entre le coeur et le point de réflexion de l'onde réfléchie, respectivement, et ledit endroit ; $\Delta T2$ une différence de temps entre le temps de référence pour l'onde d'éjection et le temps de référence pour l'onde réfléchie qui sont trouvés par l'unité de détection de temps de référence (2) ; et W2 une amplitude de l'onde pulsée qui correspond au temps de référence pour l'onde réfléchie trouvée par l'unité de détection d'amplitude d'onde pulsée (3).

2. Dispositif de mesure de vitesse d'ondes pulsées tel qu'il est revendiqué dans la revendication 1, étant précisé que l'unité de détection d'amplitude d'onde pulsée (3) contient une unité d'élimination de composante d'onde d'éjection (4) pour trouver une composante d'onde d'éjection contenue dans l'amplitude de l'onde pulsée qui correspond au temps de référence pour la composante d'onde réfléchie, éliminer la composante d'onde d'éjection à partir de l'amplitude de l'onde pulsée qui correspond au temps de référence pour la composante d'onde réfléchie, et trouver ainsi une amplitude de la composante d'onde réfléchie qui correspond au temps de référence pour la composante d'onde réfléchie, et

que l'unité de détection de vitesse d'onde pulsée (5) trouve la vitesse de l'onde pulsée sur la base du temps de référence pour la composante d'onde d'éjection et du temps de référence pour la composante d'onde réfléchie qui ont été détectés par l'unité de détection de temps de référence (2), de l'amplitude de l'onde pulsée qui correspond au temps de référence pour la composante d'onde d'éjection qui a été détectée par l'unité de détection d'amplitude d'onde (3), et de l'amplitude de la composante d'onde réfléchie qui a été trouvée par l'unité d'élimination de composante d'onde d'éjection (4), par l'équation (2) suivante au lieu de l'équation (1) :

$$\mathtt{PWV1 = d \times [(2h+d) / \alpha \times exp(\beta \times W3)]1/2 - \Delta T2] - 1 \qquad (2)}$$

dans laquelle W3 désigne une amplitude de l'onde réfléchie qui correspond au temps de référence pour l'onde réfléchie trouvée par l'unité d'élimination de composante d'onde d'éjection (4).

3. Dispositif de mesure de vitesse d'ondes pulsées tel qu'il est revendiqué dans la revendication 1, étant précisé que l'unité de détection de vitesse d'onde pulsée (5) trouve la vitesse de l'onde pulsée sur la base d'une différence de temps entre le temps de référence pour la composante d'onde d'éjection et le temps de référence pour la composante d'onde réfléchie qui ont été détectés par l'unité de détection de temps (2), et d'une différence d'amplitude entre l'amplitude de l'onde pulsée qui correspond au temps de référence pour la composante d'onde d'éjection et qui a été détectée par l'unité de détection d'amplitude d'onde pulsée (3), et l'amplitude de la composante d'onde réfléchie qui a été trouvée par l'unité d'élimination de composante d'onde d'éjection (4).

4. Programme de mesure de vitesse d'onde pulsée qui fait exécuter par un ordinateur :

une fonction de dérivée de temps de référence qui consiste à trouver un temps de référence pour l'identification d'une composante d'onde d'éjection contenue dans une onde pulsée à un endroit d'un corps vivant, et un temps de référence pour l'identification d'une composante d'onde réfléchie contenue dans l'onde pulsée ; une fonction de dérivée d'amplitude d'onde pulsée qui consiste à trouver une amplitude de l'onde pulsée qui correspond au temps de référence pour la composante d'onde d'éjection et à trouver une amplitude de l'onde pulsée qui correspond au temps de référence pour la composante d'onde réfléchie ; et une fonction de dérivée de vitesse d'onde pulsée qui consiste à calculer une vitesse de l'onde pulsée à laquelle l'onde pulsée se propage, caractérisé en ce que la fonction de dérivée de vitesse d'onde pulsée comprend le calcul de la vitesse de l'onde pulsée, sur la base du temps de référence pour la composante d'onde d'éjection, du temps de référence pour la composante d'onde réfléchie, de l'amplitude de l'onde pulsée qui correspond au temps de référence pour la composante d'onde d'éjection, et de l'amplitude de l'onde pulsée qui correspond au temps de référence pour la composante d'onde réfléchie, par l'équation (1) suivante :

$$\mathtt{PWV1 = d \times [(2h+d) / \alpha \times exp(\beta \times W2)]1/2 - \Delta T2] - 1 \qquad (1)}$$

dans laquelle PWV1 désigne une vitesse d'onde pulsée de l'onde d'éjection ; $\alpha$, $\beta$ des constantes connues définies par la relation $(PWV)^2 = \alpha \times exp(\beta \times P)$ entre la vitesse d'onde pulsée PWV et la pression artérielle P ; d, h des valeurs mesurées des distances entre le coeur et le point de réflexion de l'onde réfléchie, respectivement, et ledit endroit ; $\Delta T2$ une différence de temps entre le temps de référence pour l'onde d'éjection et le temps de référence pour l'onde réfléchie qui sont trouvés par l'unité de détection de temps de référence (2) ; et W2 une amplitude de l'onde pulsée qui correspond au temps de référence pour l'onde réfléchie trouvée par l'unité de détection d'amplitude d'onde pulsée (3).

5. Programme de mesure de vitesse d'onde pulsée tel qu'il est revendiqué dans la revendication 4, étant précisé que la fonction d'élimination de composante d'onde d'éjection (4) qui consiste à trouver une composante d'onde d'éjection contenue dans l'amplitude de l'onde pulsée qui correspond au temps de référence pour la composante d'onde réfléchie, à éliminer la composante d'onde d'éjection, contenue dans l'amplitude de l'onde pulsée, à partir de l'amplitude de l'onde pulsée qui correspond au temps de référence pour la composante d'onde réfléchie, et à trouver ainsi une amplitude de la composante d'onde réfléchie qui correspond au temps de référence pour la composante d'onde réfléchie, et que la fonction de dérivée de vitesse d'onde pulsée (5) comprend le calcul de la vitesse de l'onde pulsée sur la base du temps de référence pour la composante d'onde d'éjection, du temps de référence pour la composante d'onde réfléchie, de l'amplitude de l'onde pulsée qui correspond au temps de référence pour la composante d'onde d'éjection, et de l'amplitude de la composante d'onde réfléchie qui a été trouvée par la fonction d'élimination de composante d'onde d'éjection, par l'équation (2) suivante au lieu de l'équation (1) :

$$PWV1 = d \times [(2h+d) / \alpha \times \exp(\beta \times W3)]1/2 - \Delta T2]^{-1} \qquad (2)$$

dans laquelle W3 désigne une amplitude de l'onde réfléchie qui correspond au temps de référence pour l'onde réfléchie trouvée par l'unité d'élimination de composante d'onde d'éjection (4).

6. Programme de mesure de vitesse d'ondes pulsées tel qu'il est revendiqué dans la revendication 5, étant précisé que la fonction de dérivée de vitesse d'onde pulsée comprend l'étape qui consiste à trouver la vitesse de l'onde pulsée sur la base d'une différence de temps entre le temps de référence pour la composante d'onde d'éjection et le temps de référence pour la composante d'onde réfléchie, et d'une différence d'amplitude entre l'amplitude de l'onde pulsée qui correspond au temps de référence pour la composante d'onde d'éjection, et l'amplitude de la composante d'onde réfléchie qui a été trouvée par la fonction d'élimination de composante d'onde d'éjection.

## Fig.1

6 EJECTION AND REFLECTED WAVES INFORMATION EXTRACTION UNIT

10 PULSE WAVE VELOCITY MEASUREMENT DEVICE

## Fig.2A

*Fig.2B*

EP 2 499 967 B1

## Fig.2C

THIRD DERIVATIVE (V／SEC³)

TIME (SEC)

— THIRD DERIVATIVE
o DERIVATIVE MAXIMUM POINT
× MAXIMUM POINT
＊ REFLECTION POINT

*Fig.2D*

Fig.3

EP 2 499 967 B1

*Fig.4*

*Fig.5*

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2003010139 A
- JP 2007007075 A

- EP 1273264 A

### Non-patent literature cited in the description

- **TAKAZAWA K et al.** Underestimation of vasodilator effects of nitroglycerin by upper limb blood pressure. *Hypertension,* 1995, vol. 26, 520-3

- **MCCOMBIE, DEVIN.** Development of a wearable blood pressure monitor using adaptive calibration of peripheral pulse transit time measurements. *Ph.D. Thesis,* 2008